# EUROPÄISCHE PATENTSCHRIFT

(11) **EP 0 758 643 B1**
(45) Veröffentlichungstag und Bekanntmachung des Hinweises auf die Patenterteilung: **17.05.2000**
(21) Anmeldenummer: 96112939.2
(22) Anmeldetag: 12.08.1996
(51) Int. Cl.: C07C 255/50, C07C 253/14

(54) **Verfahren zur Herstellung von ortho-Nitro-benzonitrilen**
Process for the preparation of ortho-nitro-benzonitriles
Procédé de préparation d'ortho-nitro-benzonitriles

(30) Priorität: 16.08.1995 DE 19529973
(43) Veröffentlichungstag der Anmeldung: 19.02.1997
(73) Patentinhaber: Clariant GmbH, 65929 Frankfurt am Main (DE)
(72) Erfinder: Wessel, Thomas, Dr., 60386 Frankfurt (DE); Koch, Peter, Dr., 63179 Obertshausen (DE)

(56) Entgegenhaltungen:
- EP-A- 0 608 713
- EP-A- 0 613 719
- US-A- 4 801 728
- PATENT ABSTRACTS OF JAPAN vol. 15, no. 263 (C-0847), 4.Juli 1991 & JP 03 090057 A (ISHIHARA SANGYO KAISHA LTD), 16.April 1991,

## Beschreibung

Die vorliegende Erfindung betrifft ein Verfahren zur Herstellung von ortho-Nitrobenzonitrilen der allgemeinen Formel I, 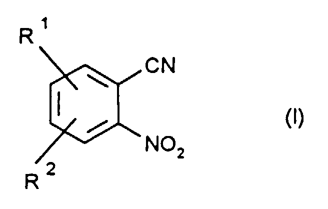 in der R¹ und R² für Wasserstoff oder elektronenziehende Gruppen stehen, durch Umsetzung der entsprechenden ortho-Fluornitrobenzole mit einem Cyanid oder einer Cyanidabgebenden Substanz.

Die Verbindungen der allgemeinen Formel I sind u. a. wichtige Zwischenprodukte für die Herstellung von Benzoesäure- und Benzoesäurealkylester-Derivaten, die wiederum wichtige Zwischenprodukte für die Herstellung von verschiedenen Wirkstoffen sind, z. B. für die Herstellung von Herbiziden wie den Verbindungen der EP-A-496 631 oder von Isoxazol-Herbiziden. Verschiedene Verfahren zur Herstellung von ortho-Nitrobenzonitrilen der allgemeinen Formel I sind bereits beschrieben. In der Regel werden dabei nach der Rosenmund-von Braun-Reaktion die entsprechenden ortho-Chlornitrobenzole mit einem Schwermetallcyanid, insbesondere Kupfer(I)cyanid, umgesetzt. In der US-A-2 195 076 werden beispielsweise Halogen-Cyan-Austauschreaktionen beschrieben, bei denen mit Kupfercyanid in Gegenwart von Stickstoffbasen wie Pyridin oder Chinolin bei hohen Temperaturen gearbeitet wird. Bei Kupfer-katalysierten Halogen-Cyan-Austauschreaktionen am Aromaten nimmt die Reaktivität des Halogenaromaten in der Reihenfolge I > Br > Cl" F deutlich ab (siehe z. B. J. Chem. Soc. 1964, 1097).

Ein wesentlicher Nachteil dieser Herstellmethode ist, daß sie den Einsatz von Schwermetallen wie Kupfer erfordert. Die Entfernung der Schwermetalle aus den Abwässern erfordert besondere Maßnahmen. Bei der Durchführung der Reaktion fallen als Nebenprodukt zwangsläufig erhebliche Mengen an Schwermetallverbindungen an, die aufwendig aufgearbeitet oder entsorgt werden müssen.

Teilweise, so z. B. bei dem in der EP-B-97 357 beschriebenen Verfahren, bei dem bei ca. 200 °C gearbeitet wird, werden bei der Herstellung von Benzonitrilen durch Halogen-Cyan-Austausch zusätzlich zum Kupfercyanid weitere Kupfersalze wie Kupfer(II)bromid eingesetzt, wodurch der Anfall an Schwermetallsalzen zusätzlich erhöht wird.

Die EP-B-497 765 offenbart ein Verfahren zur Herstellung von ortho-Nitrobenzonitrilen aus den entsprechenden ortho-Chlornitrobenzolen, bei dem stattdessen Alkali-, Erdalkali- oder Zinkbromide zugesetzt werden, da aber das eigentliche Reagenz weiterhin Kupfercyanid bzw. eine Mischung aus Kupfer(II)bromid und Lithiumcyanid ist, liefert auch diese Verfahrensweise einen hohen Anfall an Schwermetallsalzen.

Nach dem Verfahren der DE-A-2 610 675 können ortho-Cyanazo-farbstoffe aus den entsprechenden ortho-Bromazofarbstoffen erhalten werden, indem der Halogen-Cyan-Austausch unter Phasentransferbedingungen mit einer Mischung aus Kupfer- oder Zinkcyanid mit Natrium- oder Kaliumcyanid durchgeführt wird. Da aber das Schwermetallcyanid nur partiell durch das Alkalimetallcyanid substituiert werden kann, ist auch dieses Verfahren, ebenso wie das der EP-A-334 188, bei dem das Kupfercyanid partiell durch Alkalimetallcyanide ersetzt werden kann, weiterhin mit einem erheblichen Schwermetallanfall verbunden.

Ein Fluor-Cyan-Austausch am Aromaten unter Verwendung eines Alkalimetallcyanids wird in der US-A-5 386 051 und der EP-A-608 713 beschrieben. Diese Dokumente betreffen aber jeweils nur eine einzelne Substanz mit einer bestimmten Substituentenkombination, und bei der Durchführung der Verfahren werden organische Lösungsmittel eingesetzt, die bei einer Übertragung in den technischen Maßstab aufwendige apparatetechnische Maßnahmen, beispielsweise aus arbeitshygienischen Gründen oder zum Feuchtigkeitsausschluß, erfordern und ökonomisch ungünstig sind.

Obwohl ortho-Fluornitrobenzole über Chlor-Fluor-Austauschreaktionen gut zugänglich sind (siehe z. B. J. Am. Chem. Soc. 78 (1956), 6034 und i. Org. Chem. 56 (1991), 6406), wurden Fluor-Cyan-Austauschreaktionen zur Herstellung der Verbindungen der allgemeinen Formel I bislang aber kaum in Betracht gezogen. Dies ist in erster Linie darauf zurückzuführen, daß ortho-Nitrobenzonitrile bei der Herstellung aus ortho-Fluornitrobenzolen und Alkalimetallcyaniden mit den Alkalimetallcyaniden in einer Nefartigen Reaktion zu Cyanphenolen weiterreagieren (siehe J. Org. Chem. 40 (1975), 3746; vgl. auch J. Chem. Soc., Chem. Comm. 1976, 972).

Ein allgemein anwendbares, einfaches, ökonomisch günstiges Verfahren zur Herstellung der Verbindungen der allgemeinen Formel I, bei dem keine Schwermetallverbindungen anfallen, steht weiterhin nicht zur Verfügung. Überraschenderweise wurde nun gefunden, daß diese Verbindungen günstig durch Fluor-Cyan-Austausch aus den entsprechenden ortho-Fluornitrobenzolen mit einem Cyanid oder einer Cyanid-abgebenden Verbindung unter Phasentransferkatalyse erhältlich sind.

Gegenstand der vorliegenden Erfindung ist ein Verfahren zur Herstellung von ortho-Nitrobenzonitrilen der allgemeinen Formel I, 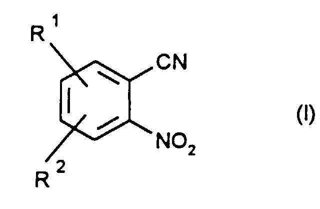 in der R¹ und R², die gleich oder verschieden sein können, für Wasserstoff oder elektronenziehende Gruppen stehen, durch Umsetzung der entsprechenden ortho-Fluornitrobenzole der allgemeinen Formel II, 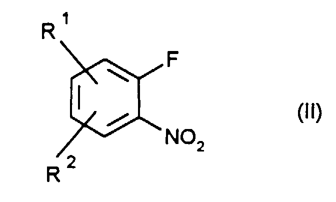 in der R¹ und R² wie für die allgemeine Formel I definiert sind, mit Alkalimetallcyaniden oder Cyanid-abgebenden Substanzen, dadurch gekennzeichnet, daß die Umsetzung in einem wäßrigen Medium in Gegenwart von Phasentransferkatalysatoren durchgeführt wird.

Beispiele für elektronenziehende Gruppen, für die R¹ und R² stehen können, sind Nitro, (C₁-C₄)-Alkylsulfonyl, Halogen-(C₁-C₄)-alkylsulfonyl, Cyano, Carboxy, ((C₁-C₄)-Alkyl)oxycarbonyl, Pentafluorethyl und durch Halogen mono-, di- oder trisubstituiertes Methyl.

Alkylgruppen können geradkettig oder verzweigt sein. Als Alkylgruppen kommen dabei beispielsweise Methyl, Ethyl, n-Propyl, i-Propyl, n-Butyl, sec-Butyl, i-Butyl und tert.-Butyl in Betracht. Bevorzugte Alkylgruppen sind Methyl und Ethyl, insbesondere Methyl.

Beispiele für Halogen sind insbesondere Fluor, Chlor und Brom. Bevorzugt steht Halogen für Fluor oder Chlor. Halogen-(C₁-C₄)-alkylsulfonyl ist beispielsweise Chlormethylsulfonyl oder 2-Chlorethylsulfonyl. Durch Halogen mono-, di- oder trisubstituiertes Methyl ist beispielsweise Chlormethyl, Dichlormethyl, Trichlormethyl, Fluormethyl, Difluormethyl oder Trifluormethyl, insbesondere Trifluormethyl.

Eine besonders bevorzugte elektronenziehende Gruppe ist die Trifluormethylgruppe.

Stehen einer oder beide der Reste R¹ und R² für elektronenziehende Gruppen, so können sich diese in beliebigen Positionen in Bezug auf die Nitrogruppe und die Cyanogruppe (bzw. die Nitrogruppe und das Fluoratom) befinden. Bevorzugt steht einer der beiden Reste R¹ und R² für Wasserstoff und der andere für eine elektronenziehende Gruppe oder beide Reste R¹ und R² für elektronenziehendeGruppen. Besonders bevorzugt steht einer der beiden Reste R¹ und R² für Wasserstoff und der andere für eine elektronenziehende Gruppe. In dieser besonders bevorzugten Ausführungsform der vorliegenden Erfindung steht darüberhinaus bevorzugt die elektronenziehende Gruppe in der para-Position zum Fluoratom in der allgemeinen Formel II bzw. zur Cyanogruppe in der allgemeinen Formel I.

Beispiele für Alkalimetallcyanide sind Lithiumcyanid, Natriumcyanid, Kaliumcyanid, Rubidiumcyanid und Caesiumcyanid, wobei Natrium- und Kaliumcyanid bevorzugt sind.

Als Cyanid-abgebende Verbindungen bevorzugt eingesetzt werden Cyanhydrine der allgemeinen Formel III, 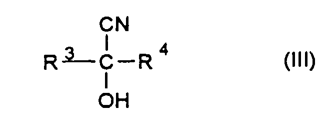 in der R³ für Wasserstoff oder eine geradkettige oder verzweigte Alkylgruppe, bevorzugt eine (C₁-C₈)-Alkylgruppe, besonders bevorzugt eine (C₁-C₄)-Alkylgruppe, steht und R⁴ für eine geradkettige oder verzweigte Alkylgruppe, bevorzugt eine (C₁-C₈)-Alkylgruppe, besonders bevorzugt eine (C₁-C₄)-Alkylgruppe, steht, die mit einer für R3 stehenden Alkylgruppe übereinstimmen oder sich von dieser unterscheiden kann. Beispiele für (C₁-C₄)-Alkylgruppen sind die oben bereits genannten Gruppen, Beispiele für (C₁-C₈)-Alkylgruppen darüberhinaus Pentyl, Hexyl, Heptyl und Octyl. Ganz besonders bevorzugt stehen R³ und R⁴ gleichzeitig für Methyl.

In dem erfindungsgemäßen Verfahren können ein oder mehrere Alkalimetallcyanide oder ein oder mehrere Cyanid-abgebende Verbindungen der allgemeinen Formel III eingesetzt werden, ebenso wie auch Gemische, die neben einem oder mehreren Alkalimetallcyaniden eine oder mehrere Verbindungen der allgemeinen Formel III enthalten.

Die erfindungsgemäße Reaktion kann sowohl in einem rein wäßrigen Medium durchgeführt werden, d. h. als Lösungs bzw. Dispergier- bzw. Verdünnungsmittel kann allein Wasser verwendet werden, oder sie kann in einem organisch-wäßrigen Medium durchgeführt werden, d. h. zusätzlich zu Wasser können auch ein oder mehrere inerte organische Lösungs- bzw. Dispergier- bzw. Verdünnungsmittel verwendet werden. Das Mengenverhältnis zwischen Wasser und organischen Lösungsmitteln kann ebenso wie die Gesamtmenge an Wasser und ggfs. Lösungsmittel in weiten Grenzen variieren und hängt vom Einzelfall ab. Die organischen Lösungsmittel können mit Wasser mischbar sein oder sich in diesem gut oder schlecht lösen. Das Reaktionsmedium kann ein- oder zweiphasig sein. Dabei kann aber auch bei Einsatz eines einphasigen Reaktionsmediums, beispielsweise auch beim Einsatz eines rein wäßrigen Mediums, in Gegenwart der Ausgangssubstanzen und/oder Produkte ein Mehrphasensystem vorliegen. Ein organisches Lösungsmittel wird vorteilhaft insbesondere dann zugesetzt, wenn die Ausgangsverbindung der allgemeinen Formel II unter den Reaktionsbedingungen nicht flüssig ist. Wird in einem wäßrig-organischen Medium gearbeitet, so sind als Lösungs- bzw. Dispergier- bzw. Verdünnungsmittel bevorzugt aprotisch-dipolare Lösungsmittel, Alkohole und unpolare Lösungsmittel. Beispiele für geeignete aprotisch-dipolare Lösungsmittel (Definition siehe z. B. Chem. Rev. 69 (1969), 1 - 32) sind N,N-Dimethylformamid, N,N-Dimethylacetamid, Dimethylsulfoxid und N-Methylpyrrolidon. Als Lösungsmittel geeignete Alkohole sind z. B. Methanol, Ethanol, n-Propanol, i-Propanol, n-Butanol oder Ethylenglykolmonomethylether.

Geeignete unpolare Lösungsmittel sind vorzugsweise aliphatische und aromatische Kohlenwasserstoffe und Kohlenwasserstoffgemische, wie Hexan, Heptan, Cyclohexan, Methylcyclohexane, Toluol, Xylol oder Benzinfraktionen, sowie chlorierte Kohlenwasserstoffe. Als Lösungsmittel können aber beispielsweise auch Ketone, wie z. B. Aceton oder Ethylmethylketon, oder Ether oder Carbonsäureester, wie z. B. Essigsäureethylester oder Essigsäurebutylester, eingesetzt werden. Besonders bevorzugt ist als Lösungsmittel Toluol. Ganz besonders bevorzugt ist es aber, die Umsetzung in einem rein wäßrigen Medium ohne Zusatz eines organischen Lösungsmittels durchzuführen.

Als Phasentransferkatalysatoren (siehe z. B. Angewandte Chemie 86 (1974), 187 oder W. P. Weber und G. W. Gokel, Phase Transfer Catalysis in Organic Synthesis, Springer Verlag Berlin, Heidelberg, New York 1977) eignen sich allgemein beispielsweise die Halogenide, wie Fluoride, Chloride, Bromide und lodide, die Cyanide, die Hydroxide, die Hydrogensulfate, die (C₁-C₄)-Alkylsulfate, insbesondere Methyl- und Ethylsulfate, oder die Tetrafluoroborate von quartären Stickstoffverbindungen und Phosphoniumverbindungen. Besonders geeignet als Phasentransferkatalysatoren sind ebenfalls Verbindungen mit Kronenethereigenschaften, wie z. B. Benzo[15]krone-5 oder [18]Krone-6-tetracarbonsäure, oder als Beispiel für einen offenkettigen Kronenether Tris-(3,6-dioxaheptyl)-amin.

Beispiele für Gruppen mit quartärem Stickstoffatom, die in den Phasentransferkatalysatoren enthalten sein können, sind Tetra-(C₁-C₄)-alkylammonium wie Tetraethyl-, Tetrapropyl-, Tributylmethyl- und Tetrabutylammonium; Tetraalkylammonium mit einem oder mehreren längerkettigen Alkylresten wie Tetrahexyl-, Tetraoctyl-, Methyltrioctyl-Hexadecyltrimethyl- und Ethylhexadecyldimethylammonium; Cycloalkylammonium wie Cyclohexyldiethyl-n-butylammonium; Aralkylammonium wie Tri-(C₁-C₄)-alkyl)benzylammonium, (C₁-C₆)-Alkylbenzyldimethylammonium, Benzyltrimethyl-Benzyltriethyl-, Benzyltributyl-, Benzylcyclohexyldiethyl-, Benzyl-di-n-propylethyl-, Benzyl-di-n-butylethyl-, Benzylbutylcyclohexylethyl-, Dibenzyl-di-n-propyl-, Dibenzyl-di-n-butyl-, Cyclohexyldibenzylethyl-, Dibutylethylphenethyl-, Benzylnonyldibutyl-, Benzyldecyldibutyl- und Benzyldodecyldimethylammonium; Ammoniumgruppen mit Hydroxy- oder Alkoxyresten wie Butyl-di-(2-methoxyethyl)-ethyl-, Di-n-butyl-di-(2-methoxyethyl)-, n-Butyl-(2-methylbutyl)-di-(2-methoxyethyl)-, Di-(2-methoxyethyl)-di-n-propyl-, Di-(2-methoxyethyl)-diamyl-, Benzyl-di-(2-methoxyethyl)-ethyl-, Benzyl-n-butyl-di-(2-methoxyethyl)-, Dibenzyl-di-(2-methoxyethyl)ammonium und N-Dodecyl-N-methylephedrinium; von Stickstoffheterocyclen abgeleitete Gruppen wie Morpholiniumionen, Pyrrolidiniumionen, Piperidiniumionen und Hexamethyleniminiumionen, N,N-Dimethylmorpholinium, N,N-Di-(n-butyl) morpholinium, N-Benzyl-N-ethylmorpholinium, N-Benzyl-N-hexylmorpholinium, N,N-Di-(n-butyl)pyrrolidinium, N-Benzyl-N-(n-butyl)pyrrolidinium, N,N-Dibenzylpyrrolidinium, N-(n-Butyl)-N-ethylpiperidinium, N-Benzyl-N-(n-butyl)piperidinium, N-Benzyl-N-(2-ethylhexyl)piperidinium, N-Benzyl-N-(n-butyl)-2-ethylpiperidinium, N-Benzyl-N-(n-butyl)-2-(n-butyl)-2-ethylpiperidinium, N,N-Dibenzyl-hexamethyleniminium, N-Benzyl-N-isobutylhexamethyleniminium, N-(n-Butyl)-N-isobutylhexamethyleniminium, N-Benzyl-N-(n-butyl)-3,3,5-trimethylhexamethyleniminium, N-Benzyl-N-(2-(n-butoxy)propyl)hexamethyleniminium und N-Benzylchininium; Gruppen mit 2 quartären Stickstoffatomen wie 1,ω-Di-ammonioalkane, 1,ω-Di(morpholin-4-ylio)-alkane, 1,ω-Di(pyrrolidin1-ylio)alkane, 1,ω-Di(piperidin-1-ylio)alkane, 1,w-Di(hexamethylenimin-1-ylio)alkane, 1,6-Di-(benzylbutylethylammonio)hexan, 1,8-Di-(N-benzylpyrrolidin-1-ylio)octan, 1,6-Di-(N-ethylpiperidin-1-ylio)hexan, 1,8-Di-(N-(n-butyl)piperidin-1-ylio)octan und 1,6-Di-(N-benzylhexamethylenimin-1-ylio)hexan.

Die als Phasentransferkatalysator einsetzbaren Phosphoniumsalze können Alkyl- und Arylgruppen enthalten. Beispiele für geeignete Phosphoniumgruppen sind Tetrabutylphosphonium, Tributylhexadecylphosphonium, Ethyltrioctylphosphonium, Butyltriphenylphosphonium und Tetraphenylphosphonium.

Die Phasentransferkatalysatoren sind kommerziell erhältlich oder können nach bekannten oder analog zu bekannten Methoden leicht hergestellt werden, z. B. durch Quartärnisierung von tertiären Aminen. Die Phasentransferkatalysatoren können als Feststoff oder als Lösung eingesetzt werden, bevorzugt werden sie in der üblicherweise erhältlichen Form eingesetzt, Ammoniumhydroxide z. B. als Lösung, und mit dem üblicherweise erhältlichen Anion. Gewünschtenfalls kann das Anion zunächst aber auch nach allgemein bekannten Methoden ausgetauscht werden. Die Umsetzung kann mit einem einzelnen Phasentransferkatalysator oder auch mit Gemischen von zwei oder mehr Phasentransferkatalysatoren durchgeführt werden, auch mit kommerziell erhältlichen technischen Gemischen solcher Verbindungen.

Bevorzugte Phasentransferkatalysatoren sind Tetra-((C₁-C₆)alkyl)ammonium- und Tri-((C₁-C₄)alkyl)benzylammoniumsalze mit Halogenid oder Hydrogensulfat als Anion sowie Tetra-((C₁-C₆)alkyl)phosphoniumsalze mit Halogenid als Anion. Besonders bevorzugt sind Tetra-(n-butyl)ammoniumsalze, insbesondere das Hydrogensulfat, Chlorid und Bromid, und Tetra-(n-butyl)phosphoniumsalze, insbesondere das Bromid. Besonders bevorzugt ist weiterhin aus der Gruppe der Phasentransferkatalysatoren mit Kronenethereigenschaften das Tris-(3,6-dioxaheptyl)amin.

Die als Ausgangssubstanzen eingesetzten ortho-Fluornitrobenzole der allgemeinen Formel II sind teilweise kommerziell erhältlich. Ggfs. können sie nach oder analog zu bekannten Vorschriften hergestellt, z. B. nach den in J. Am. Chem. Soc. 78 (1956), 6034 und J. Org. Chem. 56 (1991), 6406 beschriebenen Methoden. Auch die Verbindungen der allgemeinen Formel III sind überwiegend kommerziell erhältlich oder können nach bekannten Methoden leicht hergestellt werden.

Die Vorgehensweise bei der erfindungsgemäßen Herstellung der Benzonitrile der allgemeinen Formel I aus den Fluor-Verbindungen der allgemeinen Formel II entspricht den üblichen Techniken der organischen Chemie. Die genaue Durchführungsweise und die Reaktionsbedingungen hängen vom Einzelfall ab. Die Reihenfolge, in der die Komponenten zusammengegeben werden, ist im allgemeinen beliebig. Z. B. können alle Komponenten, die Ausgangssubstanz der allgemeinen Formel II, das Alkalimetallcyanid und/oder die Cyanid-abgebende Substanz, der Phasentransferkatalysator, das Wasser und gegebenenfalls weitere Lösungsmittel und Hilfsstoffe, in das Reaktionsgefäß gegeben werden und die Reaktion dann unter den gewünschten Bedingungen für die gewünschte Zeitdauer durchgeführt werden. Es kann aber z. B. auch die Verbindung der allgemeinen Formel II in Substanz oder zusammen mit Wasser und/oder gewünschtenfalls weiteren Lösungsmitteln im Reaktionsgefäß vorgelegt werden und hierzu in einer oder mehreren Portionen oder kontinuierlich das Alkalimetallcyanid, bevorzugt in Form einer Lösung, und/oder die Cyanid-abgebende Substanz, in Substanz oder gelöst bzw. verdünnt, gegeben werden. Der Phasentransferkatalysator kann dabei im Reaktionsgefäß vorgelegt werden oder auch zudosiert werden, sowohl separat als auch z. B. gelöst in der Alkalimetallcyanid-Lösung. Ebenso kann z. B. das Alkalimetallcyanid und/oder die Cyanid-abgebende Substanz zusammen mit dem Phasentransferkatalysator, Wasser und gewünschtenfalls weiteren Lösungsmitteln und Hilfsstoffen vorgelegt werden und hierzu die Verbindung der allgemeinen Formel II, in Substanz oder gelöst bzw. verdünnt, in einer oder mehreren Portionen oder kontinuierlich zugegeben werden. Die Reaktion wird dann üblicherweise unter Rühren bei der gewünschten Temperatur und gewünschtenfalls unter ein- oder mehrmaligem oder kontinuierlichem Zusatz von Hilfsstoffen oder auch Reaktionspartnern durchgeführt, bis der gewünschte Umsetzungsgrad erreicht ist. Außer ansatzweise, also diskontinuierlich, kann das erfindungsgemäße Verfahren aber auch kontinuierlich durchgeführt werden z. B. in Rührkesselkaskaden oder in einem Rohrreaktor.

Die Mengenverhältnisse der Komponenten hängen vom Einzelfall ab. Im allgemeinen wird, bezogen auf das ortho-Fluornitrobenzol der allgemeinen Formel II, das Alkalimetallcyanid bzw. die Cyanid-abgebende Substanz im Überschuß eingesetzt, Alkalimetallcyanide z. B. in der 1-bis 6fachen, bevorzugt in der 1,5-bis 5fachen, besonders bevorzugt in der 2- bis 4fachen molaren Menge, Cyanidabgebende Substanzen der allgemeinen Formel III z. B. in der 1-bis 6fachen, bevorzugt in der 1,5-bis 5fachen, besonders bevorzugt in der 1,5- bis 3,5fachen Menge.

Die Menge des Phasentransferkatalysators kann in weiten Grenzen variiert werden und wird üblicherweise in der 0,005- bis 2fachen molaren Menge, bezogen auf das ortho-Fluornitrobenzol der allgemeinen Formel II, eingesetzt, bevorzugt in der 0,01- bis 1fachen, besonders bevorzugt in der 0,01-bis 0,5fachen molaren Menge.

Vielfach ist es günstig, durch Zugabe von Säure oder Base einen bestimmten, vom Einzelfall abhängenden pH-Bereich einzustellen. Im allgemeinen wird die Reaktion im neutralen oder alkalischen Bereich durchgeführt, bevorzugt bei pH-Werten von 7 bis 12, besonders bevorzugt bei pH-Werten von 7,5 bis 11,5. Bei Verwendung von Alkalimetallcyaniden ist darüber hinaus ein pH-Wert von 9 bis 11, insbesondere von 10 bis 11, bei Verwendung von Cyanid-abgebenden Substanzen der allgemeinen Formel III darüber hinaus ein pH-Wert von 7 bis 10, insbesondere von 7 bis 8, bevorzugt. Ein bestimmter pH-Wert kann zu Beginn der Reaktion eingestellt werden. Durch portionsweises oder kontinuierliches Zudosieren von Säure oder Base kann der pH-Wert auch während der gesamten Durchführung der Reaktion bei einem bestimmten Wert oder in einem bestimmten Bereich gehalten werden. Für eine pH-Einstellung werden im allgemeinen die üblichen Säuren und Basen in Form einer Lösung geeigneter Konzentration eingesetzt, beispielsweise Salzsäure, Schwefelsäure, Phosphorsäure, Natronlauge oder Kalilauge. Auch kann durch Zusatz eines Puffersystems ein bestimmter pH-Bereich eingehalten werden.

Die Reaktion wird normalerweise bei Atmosphärendruck oder bei leichtem Überdruck, z. B. bei einem Überdruck bis zu 3 bar, durchgeführt, sie kann aber ebensogut bei höheren Überdrücken durchgeführt werden. Die Reaktionstemperatur liegt üblicherweise bei Werten von O °C bis 100 °C, bevorzugt von 20 °C bis 100 °C, besonders bevorzugt von 20 °C bis 90 °C, ganz besonders bevorzugt von 20 °C bis 80 °C. Die Temperatur kann auch während der Durchführung der Umsetzung geändert werden, beispielsweise kann sie zunächst bei einem niedrigeren Wert gehalten werden und zum Schluß zur Vervollständigung der Umsetzung erhöht werden. Die Reaktion kann bis zum weitgehenden Verbrauch der Ausgangsverbindungen der allgemeinen Formel II durchgeführt werden, vielfach ist es aber günstig, sie nur ablaufen zu lassen, bis ein bestimmter, vom Einzelfall abhängiger Umsetzungsgrad erreicht ist, dann das Reaktionsgemisch aufzuarbeiten und zurückgewonnene Ausgangssubstanz der allgemeinen Formel II wieder in den nächsten Ansatz einzusetzen.

Die Aufarbeitung des Reaktionsgemisches zur Isolierung der Verbindungen der allgemeinen Formel I kann auf übliche Weise nach an sich bekannten Isolierungs- bzw. Trennmethoden erfolgen, beispielsweise durch Filtration, Zentrifugation, Phasentrennung, Extraktion, Aussalzen, (Vakuum-)Destillation, Wasserdampfdestillation oder chromatographische Methoden. Es kann auch günstig sein, zunächst für die Aufarbeitung einen bestimmten pH-Wert einzustellen oder vom Prinzip der pH-Trennung Gebrauch zu machen, oder das Reaktionsgemisch zunächst mit Wasser zu versetzen oder zunächst ganz oder teilweise einzuengen oder z. B. organische Lösungsmittel ganz oder teilweise abzudestillieren. Die im Einzelfall angewandte Methode hängt z. B. von den physikalischen Eigenschaften der Verbindungen, z. B. dem Schmelz- und Siedepunkt und der Löslichkeit, ab. Bevorzugt erfolgt die Isolierung über Phasentrennung, gegebenenfalls nach Zusatz eines organischen Lösungsmittels, und Destillationsschritte, wobei insbesondere zur Trennung von nicht umgesetzter Ausgangssubstanz der allgemeinen Formel II und Produkt der allgemeinen Formel I die Kombination von Wasserdampfdestillation und anschließender Vakuumdestillation besonders bevorzugt ist. Das so auf einfache Art erhaltene Produkt der allgemeinen Formel I enthält im allgemeinen nur noch geringe Mengen an Verunreinigungen und kann ohne weitere Reinigung in Folgestufen eingesetzt werden. Gewünschtenfalls kann es auf übliche Art weiter gereinigt werden. Wurde die Reaktion nicht bis zur vollständigen Umsetzung der Ausgangssubstanz der allgemeinen Formel II durchgeführt, so können bei der Aufarbeitung wiedergewonnene Ausgangssubstanzen oder erhaltene Gemische aus Ausgangssubstanz und Produkt im allgemeinen direkt wieder in einen neuen Ansatz eingesetzt werden. Die bei der Umsetzung anfallenden Abwässer brauchen nur einer Behandlung zur Vernichtung vorhandenen Cyanids bzw. Cyanid-abgebender Substanzen unterzogen werden und können dann z. B. einer üblichen Abwasserreinigungsanlage zugeleitet werden. Da das erfindungsgemäße Verfahren den Verzicht auf Schwermetalle wie Kupfer beim Halogen-Cyan-Austausch ermöglicht, entfallen aufwendige Maßnahmen zur Schwermetallentfernung aus den Abwässern und die Entsorgung oder Wiederaufbereitung der Schwermetallabfälle.

### Beispiele

### Beispiel 1

Zu einer Lösung von 29,5 g Natriumcyanid in 200 ml Wasser werden 6 g Tetrabutylammoniumhydrogensulfat gegeben. Die erhaltene Mischung wird mit 30 ml 0,5 N Schwefelsäure vorsichtig auf einen pH-Wert von 10,5 eingestellt und dann mit 52 g (0,25 mol) 4-Fluor-3-nitrobenzotrifluorid versetzt. Der Ansatz wird auf 60 °C erwärmt und 1 Stunde bei dieser Temperatur gerührt, wobei der pH-Wert durch Zusatz von 0,05 N Schwefelsäure im Bereich 9,5 bis 10,5 gehalten wird. Die gaschromatographische Analyse des Reaktionsgemisches zeigt nach dieser Zeit einen Anteil von 57 % 2-Nitro-4-trifluormethylbenzonitril und 36% 4-Fluor-3-nitrobenzotrifluorid an.

Zur Produktisolierung werden nach dem Abkühlen auf Raumtemperatur die organische und die wäßrige Phase des Reaktionsgemisches im Scheidetrichter voneinander getrennt. Die organische Phase wird zur Abtrennung der Ausgangssubstanz wasserdampfdestilliert. Vom Destillat der Wasserdampfdestillation wird die organische Phase abgetrennt. Es werden so 18,1 g eines Gemisches erhalten, das nach gaschromatographischer Analyse zu 79 % aus 4-Fluor-3-nitrobenzotrifluorid und zu 15 % aus 2-Nitro-4-trifluormethylbenzonitril besteht und das direkt einem Folgeansatz zugeführt werden kann. Aus dem Rückstand der Wasserdampfdestillation wird nach Abtrennen des Wassers durch Vakuumdestillation über eine kurze Vigreux-Kolonne das Produkt isoliert. Es werden bei 20 mbar und einer Kopftemperatur von 148 - 150 °C 28,2 g Produkt erhalten, das nach gaschromatographischer Analyse 92% 2-Nitro-4-trifluormethylbenzonitril und 1 % 4-Fluor-3-nitro-benzotrifluorid enthält.

### Beispiel 2

Zu 10,5 g (0,05 mol) 4-Fluor-3-nitrobenzotrifluorid wird bei 65 °C unter Rühren über 1 Stunde eine Lösung von 5,9 g Natriumcyanid und 0,5 g Tetrabutylphosphoniumbromid in 50 ml Wasser getropft, die zuvor mit 5 ml 0,5 N Salzsäure auf einen pH-Wert von 10,5 gestellt wurde. Ansonsten wird die Umsetzung analog Beispiel 1 durchgeführt. Nach einer Reaktionsdauer von 4 Stunden zeigt die gaschromatographische Analyse des Reaktionsgemisches einen Anteil von 61 % 2-Nitro-4-trifluormethylbenzonitril und 33 % 4-Fluor-3-nitrobenzotrifluorid an.

### Beispiel 3

Analog zu Beispiel 2 wird zu 10,5 g (0,05 mol) 4-Fluor-3-nitrobenzotrifluorid bei 60 °C eine Lösung von 6 g Natriumcyanid und 1 g Tris-(3,6-dioxaheptyl)amin (TDA) in 40 ml Wasser getropft, die zuvor mit 5 ml 0,5 N Salzsäure auf einen pH-Wert von 10,5 gestellt wurde. Nach einer Reaktionsdauer von 6 Stunden liegen 40 % 2-Nitro-4-trifluormethylbenzonitril und 53 % Ausgangssubstanz vor.

### Beispiel 4

Eine Lösung von 12 g Natriumcyanid und 3 g Benzyltriethylammoniumchlorid in 80 ml Wasser wird vorsichtig mit 12 ml 0,5 N Salzsäure auf einen pH-Wert von 10,8 gestellt. Zu dieser Lösung wird bei 60 °C über 1 Stunde eine Mischung von 10,5 g (0,05 mol) 4-Fluor-3-nitrobenzotrifluorid und 20 ml Toluol getropft. Ansonsten wird die Reaktion analog Beispiel 1 durchgeführt. Nach einer Reaktionsdauer von 1,5 Stunden zeigt das Gaschromatogramm einen Anteil von 11 % 2-Nitro-4-trifluormethylbenzonitril und 86 % 4-Fluor-3-nitrobenzotrifluorid an.

### Beispiel 5

Eine Mischung von 63 g (0,3 mol) 4-Fluor-3-nitrobenzotrifluorid, 42 g Acetoncyanhydrin, 1 g Tetrabutylammoniumhydrogensulfat und 200 ml Wasser wird auf 80 °C erhitzt und 5 Stunden bei dieser Temperatur gerührt, wobei der pH-Wert durch Zutropfen von insgesamt 25 ml 2 N Natronlauge bei 7 bis 7,5 gehalten wird. Nach 5 Stunden liegen nach gaschromatographischer Analyse 30 % 2-Nitro-4-fluormethylbenzonitril und 65 % 4-Fluor-3-nitrobenzotrifluorid vor.

## Patentansprüche

1. Verfahren zur Herstellung von ortho-Nitrobenzonitrilen der allgemeinen Formel I, 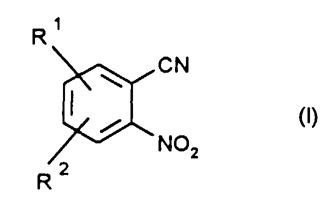 in der R¹ und R², die gleich oder verschieden sein können, für Wasserstoff oder elektronenziehende Gruppen stehen, durch Umsetzung der entsprechenden ortho-Fluornitrobenzole der allgemeinen Formel II, 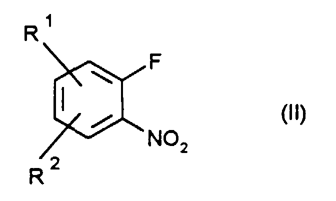 in der R¹ und R² wie für die allgemeine Formel I definiert sind, mit Alkalimetallcyaniden oder Cyanid-abgebenden Substanzen, dadurch gekennzeichnet, daß die Umsetzung in einem wäßrigen Medium in Gegenwart von Phasentransferkatalysatoren durchgeführt wird.

2. Verfahren gemäß Anspruch 1, dadurch gekennzeichnet, daß R¹ und R² unabhängig voneinander für Wasserstoff, Nitro, (C₁-C₄)-Alkylsulfonyl, Halogen-(C₁-C₄)-alkylsulfonyl, Cyano, Carboxy, ((C₁-C₄)-Alkyl)oxycarbonyl, Pentafluorethyl oder durch Halogen mono-, di- oder trisubstituiertes Methyl stehen.

3. Verfahren gemäß Anspruch 1 und/oder 2, dadurch gekennzeichnet, daß einer der Reste R¹ und R² für Wasserstoff und der andere für Trifluormethyl steht, insbesondere, daß 2-Nitro-4-trifluormethylbenzonitril hergestellt wird.

4. Verfahren gemäß einem oder mehreren der Ansprüche 1 bis 3, dadurch gekennzeichnet, daß die Umsetzung mit Natrium- oder Kaliumcyanid durchgeführt wird.

5. Verfahren gemäß einem oder mehreren der Ansprüche 1 bis 3, dadurch gekennzeichnet, daß als Cyanid-abgebende Substanzen Cyanhydrine der allgemeinen Formel III 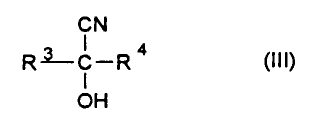 eingesetzt werden, in der R³ für Wasserstoff oder eine geradkettige oder verzweigte Alkylgruppe, bevorzugt eine (C₁-C₈)-Alkylgruppe, steht und R⁴ für eine geradkettige oder verzweigte Alkylgruppe, bevorzugt eine (C₁-C₈)-Alkylgruppe, steht, die mit einer für R³ stehenden Alkylgruppe übereinstimmen oder sich von dieser unterscheiden kann, und in der besonders bevorzugt R³ und R⁴ gleichzeitig für Methyl stehen.

6. Verfahren gemäß einem oder mehreren der Ansprüche 1 bis 5, dadurch gekennzeichnet, daß als Phasentransferkatalysatoren Salze von quartären Stickstoffverbindungen oder Phosphoniumverbindungen eingesetzt werden, bevorzugt Tetra-(n-butyl)ammoniumsalze, insbesondere das Hydrogensulfat, oder Tetra-(n-butyl)phosphoniumsalze, insbesondere das Bromid.

7. Verfahren gemäß einem oder mehreren der Ansprüche 1 bis 5, dadurch gekennzeichnet, daß als Phasentransferkatalysatoren Verbindungen mit Kronenethereigenschaften eingesetzt werden, bevorzugt Tris-(3,6-dioxaheptyl)amin.

8. Verfahren gemäß einem oder mehreren der Ansprüche 1 bis 7, dadurch gekennzeichnet, daß die Umsetzung in einem rein wäßrigen Medium ohne Zusatz eines organischen Lösungsmittels durchgeführt wird.

9. Verfahren gemäß einem oder mehreren der Ansprüche 1 bis 8, dadurch gekennzeichnet, daß die Umsetzung bei einem pH-Wert von 7 bis 12 durchgeführt wird.

10. Verfahren gemäß einem oder mehreren der Ansprüche 1 bis 9, dadurch gekennzeichnet, daß die Umsetzung bei Temperaturen von 0 °C bis 100 °C durchgeführt wird.

## Claims

1. A process for preparing ortho-nitrobenzonitriles of the formula I, 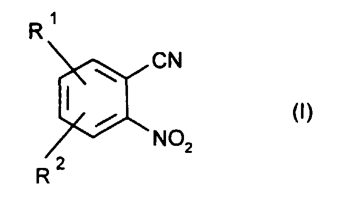 where R¹ and R², which can be identical or different, are hydrogen or electron-withdrawing groups, by reacting the corresponding ortho-fluoronitrobenzenes of the formula II, 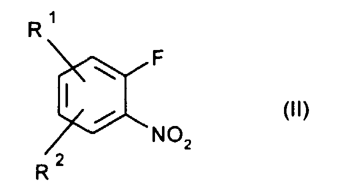 where R¹ and R² are as defined for the formula I, with alkali metal cyanides or cyanide-donating substances, wherein the reaction is carried out in an aqueous medium in the presence of phase transfer catalysts.

2. The process as claimed in claim 1, wherein R¹ and R² are, independently of one another, hydrogen, nitro, (C₁-C₄)-alkylsulfonyl, halo-(C₁-C₄)-alkylsulfonyl, cyano, carboxy, ((C₁-C₄)-alkyl)oxycarbonyl, pentafluoroethyl or methyl monosubstituted, disubstituted or trisubstituted by halogen.

3. The process as claimed in claim 1 and/or 2, wherein one of the radicals R¹ and R² is hydrogen and the other is trifluoromethyl, in particular the compound prepared is 2-nitro-4-trifluoromethylbenzonitrile.

4. The process as claimed in one or more of claims 1 to 3, wherein the reaction is carried out using sodium or potassium cyanide.

5. The process as claimed in one or more of claims 1 to 3, wherein cyanide-donating substances used are cyanohydrins of the formula III 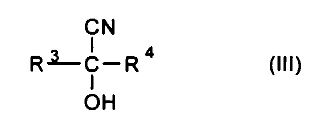 where R³ is hydrogen or a straight-chain or branched alkyl group, preferably a (C₁-C₈)-alkyl group, and R⁴ is a straight-chain or branched alkyl group, preferably a (C₁-C₈)-alkyl group, which can be the same as an alkyl group R³ or can be different therefrom, and where R³ and R⁴ are particularly preferably both methyl.

6. The process as claimed in one or more of claims 1 to 5, wherein the phase transfer catalysts used are salts of quaternary nitrogen compounds or phosphonium compounds, preferably tetra(n-butyl)ammonium salts, in particular the hydrogensulfate, or tetra(n-butyl)phosphonium salts, in particular the bromide.

7. The process as claimed in one or more of claims 1 to 5, wherein the phase transfer catalysts used are compounds having crown ether properties, preferably tris (3,6-dioxaheptyl)amine.

8. The process as claimed in one or more of claims 1 to 7, wherein the reaction is carried out in a purely aqueous medium without addition of an organic solvent.

9. The process as claimed in one or more of claims 1 to 8, wherein the reaction is carried out at a pH of from 7 to 12.

10. The process as claimed in one or more of claims 1 to 9, wherein the reaction is carried out at temperatures of from 0°C to 100°C.

## Revendications

1. Procédé de préparation d'ortho-nitrobenzonitriles de formule générale I 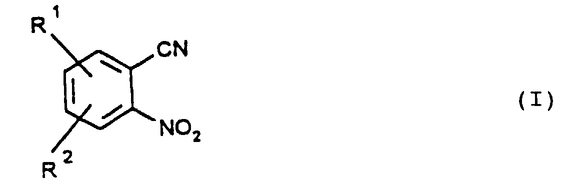 dans laquelle R¹ et R², pouvant être identiques ou différents, représentent des atomes d'hydrogène ou des groupes qui attirent les électrons, par réaction des ortho-fluoronitrobenzènes correspondants de formule générale II 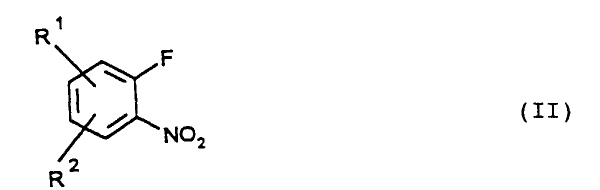 dans laquelle R¹ et R² sont définis comme à la formule générale I, avec des cyanures de métaux alcalins ou des substances qui cèdent du cyanure, caractérisé en ce qu'on met en oeuvre la réaction dans un milieu aqueux en présence de catalyseurs de transfert de phase.

2. Procédé selon la revendication 1, caractérisé en ce que R¹ et R² représentent, indépendamment l'un de l'autre, des groupes nitro, (alkyl en C₁-C₄)sulfonyle, (haloalkyl en C₁-C₄)sulfonyle, cyano, carboxy, (alkyl en C₁-C₄)oxycarbonyle, pentafluoréthyle ou méthyle mono- ou trisubstitué par des atomes d'halogènes.

3. Procédé selon la revendication 1 et/ou 2, caractérisé en ce que l'un des restes R¹ et R² représente un atome d'hydrogène et l'autre représente un groupe trifluorométhyle, en particulier, en ce qu'on prépare le 2-nitro-4-trifluorométhylbenzonitrile.

4. Procédé selon une ou plusieurs des revendications 1 à 3, caractérisé en ce qu'on met en oeuvre la réaction en utilisant le cyanure de sodium ou de potassium.

5. Procédé selon une ou plusieurs des revendications 1 à 3, caractérisé en ce qu'on emploie, en tant que substances qui cèdent du cyanure, des cyanhydrines de formule générale III 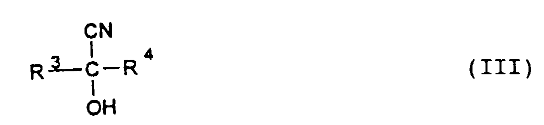 dans laquelle R³ représente un atome d'hydrogène ou un groupe alkyle linéaire ou ramifié, de préférence un groupe alkyle en C₁-C₈, et R⁴ représente un groupe alkyle linéaire ou ramifié, de préférence un groupe alkyle en C₁-C₈, lequel groupe alkyle peut être identique ou différent d'un groupe alkyle représenté par R³, et dans laquelle R³ et R⁴ représentent de manière particulièrement préférée simultanément méthyle.

6. Procédé selon une ou plusieurs des revendications 1 à 5, caractérisé en ce qu'on emploie, en tant que catalyseurs de transfert de phase, des sels de composés azotés quaternaires ou de composés de phosphonium, de préférence des sels de tétra(n-butyl)ammonium, en particulier le bisulfate, ou des sels de tétra(n-butyl)phosphonium, en particulier le bromure.

7. Procédé selon une ou plusieurs des revendications 1 à 5, caractérisé en ce qu'on emploie en tant que catalyseurs de transfert de phase des composés possédant des propriétés des éthers couronnes, de préférence la tris-(3,6-dioxaheptyl)amine.

8. Procédé selon une ou plusieurs des revendications 1 à 7, caractérisé en ce que la réaction est mise en oeuvre dans un milieu purement aqueux sans ajout de solvant organique.

9. Procédé selon une ou plusieurs des revendications 1 à 8, caractérisé en ce que la réaction est mise en oeuvre à un pH de 7 à 12.

10. Procédé selon une ou plusieurs des revendications 1 à 9, caractérisé en ce que la réaction est mise en oeuvre à une température de 0°C à 100°C.
